# EUROPEAN PATENT APPLICATION

(11) **EP 4 082 517 A1**
(43) Date of publication of application: **02.11.2022**
(21) Application number: 21171696.4
(22) Date of filing: 30.04.2021
(51) Int. Cl.: A61K 8/27, A61K 8/34, A61K 8/37, A61K 8/40, A61Q 19/00, A61Q 17/00

(54) **COMPOSITION COMPRISING A ZINC SALT AND AN ADDITIONAL EFFECTIVE INGREDIENT**

(71) Applicant: Cosphatec GmbH, 20457 Hamburg (DE)
(72) Inventor: Dietz, Carsten, Hamburg (DE); Schröder, Markus, Hamburg (DE); Scholz, Jonas, Hamburg (DE); Wientapper, Johanna, Hamburg (DE); Brauns, Frank, Hamburg (DE)
(74) Representative: Uexküll & Stolberg

(57) **Abstract**

The present invention relates to compositions comprising at least one zinc salt and at least one additional effective ingredient in an amount achieving a synergistical antimicrobial effect, wherein the additional effective ingredient is selected from glyceryl esters of C₈ to C₁₂ carboxylic acids, C₃ to C₁₀ vicinal diols, C₆- to C₁₀ hydroxamic acids, bi-(hydroxyphenyl) compounds, and/or mixtures thereof, and to methods of inhibiting microbial growth in a composition using such combination as well as to the use of the combination for controlling microbial growth in a composition.

## Description

### Field of the Invention

The present invention relates to compositions comprising at least one zinc salt and at least one additional (active) ingredient effective for inhibiting microbial growth in the compositions as well as methods of inhibiting microbial growth in compositions such as cosmetic compositions. Further, the invention relates to the use of at least one zinc salt and at least one additional effective ingredient for inhibiting microbial growth.

### Background of the invention

Microbial contamination of cosmetic products is problematic and is a major cause of product recall and industry economic loss. Cosmetic products are ideal substrates for the survival and development of a large variety of microorganisms, since they contain water and many nutrients that facilitate their growth.

The presence of certain microorganisms in cosmetic products can pose a health risk for consumers, for example in the form of skin irritation, allergic contact dermatitis and infection, especially in the eyes, mouth or wounds. Moreover, the presence of microorganisms can negatively affect the organoleptic properties, e.g., the smell, viscosity and colour, of cosmetic products.

The use of preservatives that are able to reduce microbial load to acceptable levels has improved the microbiological quality of cosmetics. However, customer attention towards ingredients contained in consumer products is growing and preservatives in personal care products are often criticized by customers. In addition, official regulations and obligations on ingredient declaration are becoming more extensive and more detailed. Economic viability of companies offering policies and labels for product evaluation regarding safety and environmental aspects demonstrate a market demand for more transparency, safety and sustainability.

Customer awareness in combination with rating systems results in pressure on manufacturers to remove criticized raw materials from the ingredient portfolio available for production. At the same time this creates demand for other materials and new solutions to compensate for avoided preservatives.

One solution to compensate for a constrained use of criticized preservatives is to complement or replace classical systems by other ingredients providing antimicrobial properties in addition to their main function. Since efficiencies of those ingredients can vary in strength and in regard to the microbial spectrum they influence, combinations have to be well selected and balanced to achieve adequate preservation of a formulation.

Zinc compounds like zinc salts of organic acids or also zinc oxide have been used for antimicrobial purposes in a variety of industrial applications ranging from food to pharmaceuticals as well as personal care. While antimicrobial properties of zinc are well described and undisputed, the underlying mechanisms are still a matter of discussion and research and might differ between compounds and applications.

Zinc inhibits enzymes which produce free radicals and inflammatory mediators. It is able to reduce odors of bacterial origin and disrupts the cell equilibrium of acne causing bacteria. Furthermore, zinc is an essential trace element. On one hand, a deficiency of zinc can lead to severe consequences for a variety of biological functions. On the other hand, an excess of zinc can also have a negative impact on physiological systems. This is the reason why food supplements as well as personal care regulations include recommendations and limitations for the content of zinc. The use of zinc is restricted to a maximum of 1 % in personal care formulations by the European Cosmetics Regulation (Regulation (EC) *No 1223*/*2009*). The beneficial use of zinc compounds for antimicrobial purposes should therefore be well dosed under consideration of the corresponding guidelines. Application concentrations of zinc for microbial growth control should be kept reasonably low which makes it advisable to augment their efficiency by complementary mechanisms.

WO 2007/076125 A2 relates to antimicrobial compositions comprising at least 0.05 wt% of a zinc compound. The compositions are described to be non-toxic to mammals and plants and effective against a broad spectrum of detrimental pathogenic microorganisms.

Developers are increasingly searching for strategies to apply multifunctional raw materials with antimicrobial secondary functions to gain an adequate protection of their formulation against the full spectrum of microbial growth while avoiding the use of traditional preservatives. New solutions for microbial growth control in compositions avoiding classical preservatives are needed. Hence there is a demand for novel combinations of antimicrobial agents having a high efficacy of controlling the growth of microorganisms.

In particular, there is a need to find new solutions for microbial growth control of personal care formulations or cosmetic compositions avoiding classical preservatives.

### Summary of the invention

According to the present invention the above problems are solved by a composition comprising at least one zinc salt and at least one additional effective ingredient in an amount achieving a synergistic antimicrobial effect, wherein the additional effective ingredient is selected from:
- glyceryl esters of C₈- to C₁₂ carboxylic acids,
- C₃ to C₁₀ vicinal diols,
- C₆ to C₁₀ hydroxamic acids,
- bi-(hydroxyphenyl) compounds and/or
- mixtures thereof.

It has been surprisingly found that compositions comprising at least one zinc salt and at least one additional effective ingredient selected from glyceryl esters of C₈ to C₁₂ carboxylic acids, C₃ to C₁₀ vicinal diols, C₆ to C₁₀ hydroxamic acids, and bi-(hydroxyphenyl) show a synergistic antimicrobial effect. Thus, low amounts of zinc can be used in the composition to achieve good microbial growth control. Thus, despite the overall level of antimicrobial agent being low, adequate protection against microorganisms can be achieved in the compositions of the present invention.

This is in particular beneficial for cosmetic compositions and formulations because higher skin tolerance can be achieved. Based on the European Cosmetic Regulation (No 1223/2009) the amount of zinc in cosmetic formulations is limited. The potential of zinc salts in lower dosages can be used by adding the additional effective ingredients. The combination of the zinc salt and at least one additional effective ingredient is surprisingly effective in reducing or preventing growth or proliferation of microorganisms in compositions in accordance with the present invention.

The invention also provides methods of inhibiting microbial growth in a composition as defined in more detail hereinbelow.

Finally, the present invention provides the use of at least one zinc salt and at least one additional effective ingredient for controlling microbial growth.

Further aspects of the invention are disclosed in the appended claims. In certain aspects of the invention, embodiments consist of the components disclosed herein.

The invention also relates to the combination of the at least one zinc salt and at least one additional effective ingredient in an amount achieving a synergistic antimicrobial effect in a composition to which the combination is added.

### Detailed description of the invention

According to the present invention compositions are provided which comprise at least one zinc salt and at least one additional effective ingredient in an amount achieving a synergistic antimicrobial effect, wherein the additional effective ingredient is selected from:
- glyceryl esters of C₈- to C₁₂ carboxylic acids,
- C₃ to C₁₀ vicinal diols,
- C₆ to C₁₀ hydroxamic acids,
- bi-(hydroxyphenyl) compounds, and/or
- mixtures thereof.

In the context of the present application, an antimicrobial effect is the effect of the agent of reducing the growth or proliferation of and/or killing microorganisms such as bacteria and fungi in a composition comprising the agent.

The composition of the present invention comprises at least one zinc salt and at least one additional effective ingredient in an amount achieving a synergistic antimicrobial effect. Thus, the antimicrobial effect of the combination of the at least one zinc salt and at least one additional effective ingredient is larger then the antimicrobial effect of the sum of the effects of each of the ingredients when used alone in the respective concentrations.

Accordingly, the reduction of the number of viable bacteria, yeast and/or mould is larger for the combination of the at least one zinc salt and at least one additional effective ingredient compared to the sum of the reduction of the number of viable bacteria achieved by the addition of the identical amounts of the individual components. Thus, the combination has an antimicrobial effect beyond the sum of the effects of each component of the combination taken in isolation.

The antimicrobial effect can be determined as described in the examples section. In this case, a composition only containing the components of the composition of the present invention as active antimicrobial agents should be considered.

The term "antimicrobial agent" is intended to mean an agent which provides an antimicrobial benefit as would be understood by a person skilled in the art. For example, an agent that is capable of preventing or reducing the growth or proliferation of and/or killing microorganisms such as bacteria and fungi.

The composition may comprise the zinc salt and the additional effective ingredient in a ratio of 15:1 to 1:3, preferably between 10:1 and 1:2.

In a preferred embodiment of the invention, the composition contains one zinc salt and one additional effective ingredient described herein only.

The composition of the present invention comprises the combination of at least one zinc salt and at least one additional effective ingredient.

In a preferred embodiment of the invention, the composition comprises the combination of one zinc salt and one additional effective ingredient as defined herein.

### Zinc salts

The composition of the present invention comprises at least one zinc salt.

In a preferred embodiment of the invention, the at least one zinc salt is selected from the group consisting of zinc lactate, zinc L-ascorbate, zinc L-aspartate, zinc L-lysinate, zinc mono L-methioninsulfate, zinc sulfate, zinc bisglycinate, zinc chloride, zinc citrate, zinc malate, zinc carbonate, zinc L-pidolate, zinc picolinate, zinc acetate and/or mixtures thereof.

Preferably, the zinc salt is a zinc salt of a carboxylic acid, in particular a C₃ to C₁₀ carboxylic acid, preferably a C₃ to C₁₀ or C₃ to C₆ carboxylic acid having one or more hydroxyl groups.

In a particularly preferred embodiment of the invention, the at least one zinc salt is a zinc salt of an alpha hydroxy carboxylic acid ("alpha hydroxy acid"), which may have a number of C atoms as defined above, in particular zinc lactate.

In another preferred embodiment of the invention, the at least one zinc salt is a zinc salt of an amino acid.

### Additional effective ingredient

In addition, the composition of the present invention comprises at least one additional effective ingredient.

The at least one additional effective ingredient may be a specific chemical compound or a mixture of different compounds described herein below. The at least one additional effective ingredient may also be an extract from a natural source.

In a preferred embodiment of the invention, the additional effective ingredient is an amphiphilic compound.

The additional effective ingredient may be capable of weakening the outer shell of the cell membrane structure in microorganisms.

The additional effective ingredient may be a glyceryl ester of a C₈ to C₁₂ carboxylic acid. In a preferred embodiment of the invention, the additional effective ingredient is a glyceryl monoester. The additional effective ingredient may be a glyceryl monoester of a linear C₈ to C₁₂ carboxylic acid. In a particularly preferred embodiment, the additional effective ingredient is glyceryl caprylate, glyceryl laurate or glyceryl undecylenate.

The additional effective ingredient may also be a C₃ to C₁₀ vicinal diol. In a vicinal diol, the two hydroxyl groups occupy vicinal positions, that is, they are attached to adjacent atoms. The C₃ to C₁₀ vicinal diol may be 1,2-propanediol, 1,2-butanediol, 1,2- pentanediol, 1,2-hexanediol, 1,2-heptanediol, 1,2-octanediol, 1,2-nonanediol or 1,2-decanediol.

In a preferred embodiment of the invention, the additional effective ingredient is a C₅ to C₇ vicinal diol. In a particularly preferred embodiment, the additional effective ingredient is 1,2-pentanediol, or 1,2-hexanediol.

The additional effective ingredient may also be a C₆ to C₁₀ hydroxamic acid. A hydroxamic acid is an organic compound bearing the functional group RC(O)N(OH)R', with R and R' as organic residues and CO as a carbonyl group. In a preferred embodiment of the invention, the additional effective ingredient is a C₆- to C₁₀ hydroxamic acid with a saturated alkyl chain. In a particularly preferred embodiment, the additional effective ingredient is caprylhydroxamic acid.

The additional effective ingredient may also be at least one bi-(hydroxyphenyl) compound. In a preferred embodiment of the invention, the additional effective ingredient is at least one bi-(hydroxyphenyl) compound, wherein each phenyl ring is substituted with one allyl group. The additional effective ingredient may be Magnolol and/or Honokiol. These are polyphenolic containing compounds in which Honokiol is an isomer of Magnolol. Honokiol and Magnolol can be isolated or purified from Magnolia extracts (e.g., flower, bark, and seed cone extracts) or other extracts that include such compounds by standard techniques.

In a preferred embodiment of the invention, the additional effective ingredient is an extract comprising Magnolol and Honokiol. The extract may comprise Magnolol and Honokiol in a ratio of approximately 1:1.

The extract may be a Magnolia extract. The Magnolia extract can be obtained or derived from a variety of sources from a Magnolia plant (e.g., flower, bark, seed cone, etc.). The Magnolia extract can be obtained from the species within the *Magnoliaceae* family. Non-limiting examples of these species include *Magnolia acuminata, Magnolia ashei, Magnolia biondii, Magnolia cylindrical Magnolia cambellii, Magnolia denudata, Magnolia fraseri, Magnolia grandiflora, Magnolia hypoleuca, Magnolia kobus, Magnolia liliflora, Magnolia loegneri, Magnolia macrophylla, Magnolia officinalis, Magnolia pyramidata, Magnolia sargentiana, Magnolia seiboldii, Magnolia soulangiana, Magnolia sprengeri, Magnolia stellata, Magnolia tripetala, Magnolia virginiana, Magnolia zenii, and Michelia figo.*

In a particularly preferred embodiment, the additional effective ingredient is a *Magnolia officinalis* bark extract. The extract may be obtained via extraction with supercritical CO₂. Ethanol may be used at the end of the extraction process for purification.

In a preferred aspect of the invention, the additional effective ingredient is selected from:
- glyceryl monoester of a linear C₈ to C₁₂ carboxylic acid,
- C₅ to C₇ vicinal diol,
- C₆ to C₁₀ hydroxamic acid with a saturated alkyl chain, and/or
- an extract comprising Magnolol and/or Honokiol.

In a preferred aspect of the invention, the composition of the present invention comprises a zinc salt of an alpha hydroxy acid and an additional effective ingredient selected from
- glyceryl monoester of a linear C₈ to C₁₂ carboxylic acid,
- C₅ to C₇ vicinal diol,
- C₆ to C₁₀ hydroxamic acid with a saturated alkyl chain, and/or
- an extract comprising Magnolol and/or Honokiol.

In a preferred aspect of the invention, the composition of the present invention comprises a zinc salt of an alpha hydroxy acid and an additional effective ingredient selected from
- glyceryl monoester of a linear C₈ to C₁₀ carboxylic acid,
- C₅ to C₆ vicinal diol,
- C₆ to C₈ hydroxamic acid with a saturated alkyl chain, or
- an extract comprising Magnolol and/or Honokiol.

In a particularly preferred aspect of the invention, the composition of the present invention comprises zinc lactate and an additional effective ingredient selected from glyceryl caprylate, 1,2-pentanediol, 1,2-hexanediol, caprylhydroxamic acid or a *Magnolia officinalis* bark extract.

In another particularly preferred aspect of the invention, the composition of the present invention comprises zinc lactate and an additional effective ingredient selected from glyceryl caprylate, 1,2-pentanediol, caprylhydroxamic acid or a *Magnolia officinalis* bark extract.

### Composition of the invention

The composition of the present invention may be a topical skin care composition. The composition of the present invention may also be a cosmetic composition. The composition may be for topical use on a subject. The composition of the present invention may further be a personal care formulation. The composition of the present invention is suitable for, but not limited to, formulations for use on lips, nails, skin, eyelashes, eyebrows, teeth or hair. In another embodiment, the composition is an oral care formulation.

In a preferred embodiment, the zinc salt is present in the composition of the present invention in an amount of more than 0.01 wt%, preferably more than 0.1 wt%, relative to the total weight of the composition. In a preferred embodiment, the zinc salt is present in an amount of 0.01 wt% to 4 wt%, relative to the total weight of the composition. In a particularly preferred embodiment, the zinc salt is present in an amount of 0.3 wt% to 4 wt%, in particular 0.5 to 1 wt%.

In a preferred embodiment, the additional effective ingredient is present in the composition of the present invention in an amount of 0.01 wt% to 2 wt%, relative to the total weight of the composition. In a particularly preferred embodiment, the additional effective ingredient is present in an amount of 0.03 wt% to 1.5 wt%.

The combination of at least one zinc compound and at least one additional effective ingredient may be present in the composition in an amount of more than 0.02 wt%, preferably more than 0.1 wt%, more preferably more than 0.4 wt%, relative to the total weight of the composition.

In a preferred embodiment, the combination is present in an amount of from 0.02 wt% to 6 wt%, relative to the total weight of the composition, preferably from 0.05 to 5 wt%, from 0.1 to 3 wt% or from 0.5 to 2 wt%.

The combination of at least one zinc salt and at least one additional effective ingredient may be present in the composition in an amount effective to reduce the number of viable/live bacteria, yeast and/or mould such as *Enterobacter gergoviae, Pseudomonas aeruginosa, Staphylococcus aureus, Escherichia coli, Candida albicans,* and *Aspergillus brasiliensis.*

The combination of at least one zinc salt and at least one additional effective ingredient may be present in the composition in an amount effective to achieve an 'A criteria pass' for bacteria, yeast and mould (e.g., *Pseudomonas aeruginosa, Staphylococcus aureus, Escherichia coli, Candida albicans,* and *Aspergillus brasiliensis)* in accordance with the pass acceptance criteria outlined in the EU Pharmacopeia, section 5.1.3, and also discussed in the Examples section below.

The combination of at least one zinc salt and at least one additional effective ingredient may be present in the composition in an amount effective to achieve at least a 2 log reduction in the number of viable/live bacteria against the value obtained for the inoculum at a time point of 2 days after inoculation of the cosmetic composition and/or an amount effective to produce at least a 3 log reduction in the number of viable/live bacteria against the value obtained for the inoculum at a time point of 7 days after inoculation of the cosmetic composition.

In general, the compositions of the present invention can be formulated over a broad range of pH values, such as from 3 to 8. In one embodiment the pH can be in the range from 4 to 8, such as from 5 or 5.5 to 7. In another embodiment, the compositions can be formulated to have a pH of 6 or higher. In a particular embodiment, the compositions can be formulated to have a pH from 6 to 8, or 6.2 to 7.

The composition can be in a dry, powdered, liquid, solid, semi-solid, spray, or aerosol form.

The composition can be comprised in a cosmetic carrier (e.g., an emulsion, cream, lotion, solution, anhydrous base, gel, ointment, *etc.*). The carrier may be water- based, oil-based, or emulsion-based.

In embodiments where the carrier is emulsion-based, the composition may be in the form of a water-in-oil, an oil-in-water, a water-in-oil-in-water or an oil-in-water-in-oil emulsion.

In embodiments where the carrier is water-based, water may be present at a level of 40 wt% or more, 50 wt% or more, 60 wt% or more, 70 wt% or more, or 90 wt% or more, relative to the total weight of the composition.

The cosmetic composition of the invention may be provided in any form suitable for topical application to the skin and/or hair. The composition of the invention may be delivered and/or applied to the skin and/or hair via any of the conventional formulations known to those skilled in the art. Typical formulation types of the present invention are liquids, creams, lotions, milks, mousses, gels, sprays, serum, foams, aerosols, and ointments.

In embodiments where the composition is in the form of a water-in-oil emulsion, water may be present at a level of 20 to 60 wt%, relative to the total weight of the composition. In embodiments where the composition is in the form of an oil-in-water emulsion, water may be present in an amount of 40% to 90% wt%.

The composition of the invention will generally further comprise other ingredients or excipients which will be well known to those skilled in the art.

The composition of the invention may further comprise ingredients such as surfactants, preservatives, absorbents, adsorbents, chelating agents, lubricants, solvents, moisturizers, water repellents, anti-oxidants, UV absorbers, anti-irritants, vitamins, trace metals, antimicrobial agents and/or structuring agents.

The compositions of the invention may further comprise one or more perfumes and/or colourings.

### The method and use

In another aspect of the present invention, a method of inhibiting microbial growth in a composition is provided.

The method of inhibiting microbial growth in a composition comprises the steps of:
- introducing at least one zinc salt and at least one additional effective ingredient in an amount achieving a synergistic antimicrobial effect, wherein the additional effective ingredient is selected from glyceryl esters of C₈- to C₁₂ carboxylic acids, C₃- to C₁₀ vicinal diols, C₆- to C₁₀ hydroxamic acids, bi-(hydroxyphenyl) compounds, and/or mixtures thereof, and
- controlling the growth of at least one microorganism.

In a preferred embodiment, the composition is a cosmetic composition.

In a preferred embodiment, the at least one microorganism is a bacterium from the genus Escherichia, Pseudomonas and/or Staphylococcus, such as *Escherichia coli (E. coli),* Pseudomonas *aeruginosa (P. aeruginosa)* and/or Staphylococcus *aureus (S. aureus).*

In a preferred embodiment, the method includes introducing a zinc salt in an amount of 0.01 wt% to 4 wt% and an additional effective ingredient in an amount of 0.01 wt% to 2 wt%, relative to the total weight of the composition. In a particularly preferred embodiment, the zinc salt is introduced into the composition in an amount of 0.3 wt% to 1 wt%, in particular 0.5 to 1 wt% and the additional effective ingredient in an amount of 0.03 wt% to 1.5 wt%, relative to the total weight of the composition.

In yet another aspect of the present invention, there is provided the use of a combination of at least one zinc salt and at least one additional effective ingredient in an amount achieving a synergistic antimicrobial effect for controlling microbial growth, wherein the additional effective ingredient is selected from:
- glyceryl esters of C₈ to C₁₂ carboxylic acids,
- C₃ to C₁₀ vicinal diols,
- C₆ to C₁₀ hydroxamic acids,
- bi-(hydroxyphenyl) compounds, and/or
- mixtures thereof.

In a preferred embodiment, the composition is a cosmetic composition.

The skilled person will understand that optional features of one embodiment or aspect of the invention may be applied to, where appropriate, to other embodiments or aspects of the invention.

In particular, the specific embodiments described above for the composition of the present invention are also applicable to the method of inhibiting microbial growth in a composition and the use of at least one zinc salt and at least one additional effective ingredient for controlling microbial growth as defined above.

### Examples

The invention is illustrated by the following examples, which describe in detail the testing of the antimicrobial efficiency for a variety of different compositions of the present invention. These examples should not be considered as limiting the scope of the invention, but as illustrating it.

The testing method used is based on the Ph. Eur. chapter 5.1.3. (Ph. Eur. 8.6, 01/2011:50103) testing which is a commonly used method for testing the antimicrobial efficiency in cosmetic products.

To count the viable microorganisms in the inoculated products, the agar medium used for the initial cultivation of the respective micro-organisms was used. A series of containers of the product to be examined, was inoculated each with a suspension of one of the test organisms to give an inoculum of 10⁵ to 10⁶ micro-organisms per millilitre or per gram of the preparation. The volume of the suspension of inoculum did not exceed 1 per cent of the volume of the product. The suspension was thoroughly mixed to ensure homogeneous distribution. The inoculated product was maintained at 20-25°C, protected from light. A suitable sample was removed from each container, typically 1 ml or 1 g, at zero hour and at appropriate intervals according to the type of the product. The number of viable micro-organisms by plate count or membrane filtration was determined. It was ensured that any residual antimicrobial activity of the product was eliminated. The procedure was validated to verify its ability to demonstrate the required reduction in count of viable micro-organisms.

The formulations were inoculated with one ore more different microorganisms *(E. coli, P. aeruginosa* and *S. aureus).*

The bacterial strains used for the experiments were:
*Escherichia coli:* ATCC 8739
*Pseudomonas aeruginosa:* ATCC 9027
*Staphylococcus aureus:* ATCC 6538

Microbial count reductions of bacterial strains added to an oil-in-water emulsion were measured after 2 and/or 7 days for a variety of combinations. Synergistic antimicrobial activity is achieved if the observed microbial count reduction of a combination is higher than the sum of the reductions reached by the single components of the combination alone.

Several combinations of effective ingredients in combination with zinc lactate were tested. The combined substances were Cosphaderm^{®} Zinc Lactate natural (Zinc Lactate), Cosphaderm^{®} GMCY (Glyceryl Caprylate), Cosphaderm^{®} Pentiol natural (Pentylene Glycol), Cosphaderm^{®} CHA (Caprylhydroxamic acid), Cosphaderm^{®} Magnolia Extract 98 (Magnolia officinalis bark extract). For all tests the following oil-in-water emulsion was used (Table 2). The pH of the emulsion was 6.5.

High water content (76 %) was used in order to impede the bacteria reduction.

**Table 2:**

| **Phase** | **Ingredient** | **INCI** | **Supplier** | **wt%** |
|---|---|---|---|---|
| A | Demin. Water | Aqua | - | ad 100 |
| A | Glycerin | Glycerin | Cremer GmbH | 3.00 |
| A | Cosphaderm^{®} X 34 | Xanthan Gum | Cosphatec GmbH | 0.50 |
| B | Cosphaderm^{®} Feel | Triheptanoin | Cosphatec GmbH | 5.00 |
| B | Imwitor^{®} 372P | Glyceryl Stearate Citrate | IOI Oleo GmbH | 2.50 |
| B | Witarix^{®} 60/40 | Caprylic/Capric Triglyceride | IOI Oleo GmbH | 5.00 |
| B | Softisan^{®} 154 | Hydrogenated Palm Oil | IOI Oleo GmbH | 1.00 |
| B | Shea Butter refined | Butyrospermum Parkii Butter | Cosphatec GmbH | 5.00 |
| B | Ecorol^{®} 16/98 P | Cetyl Alcohol | Ecogreen Oleochemicals | 2.00 |

As a reference, the single effective ingredients as well as zinc lactate were added separately to the oil-in-water emulsion. In addition, a combination of effective ingredient and zinc lactate was tested. The results of these combinations have been checked for synergistic effects. All tests were evaluated together in order to have the same inoculation concentration and conditions for all samples.

The inoculation concentrations for all combinations and their references (single substances) are shown in Table 3. The abbreviation CFU stands for colony forming unit

**Table 3:**

| **pH 6.5** | | **Maximum possible reduction of bacteria** | |
|---|---|---|---|
| | **Inoculation concentration [CFU/g]** | **[CFU/g]** | **Log reduction** |
| *E. coli* | 540 000 | 0 | 5.73 |
| *P. aeruginosa* | 340 000 | 0 | 5.53 |
| *S. aureus* | 320 000 | 0 | 5.51 |

An oil-in-water emulsion comprising the combination of zinc lactate and glyceryl caprylate was incubated with *E. coli* and *P. aeruginosa* and the microbial count reduction was measured after 2 and 7 days. The reduction of colony forming units of bacteria given in logarithmic scale is given in Table 4. The expected log reductions are the sum of the log reductions of both single substances.

**Table 4:**

| **Day** | **bacteria** | **0.5 wt% zinc lactate** | **0.2 wt% glyceryl caprylate** | **0.5 wt% zinc lactate + 0.2 wt% glyceryl caprylate** | |
|---|---|---|---|---|---|
| | | | | **expected summation of log reduction** | **measured log reduction** |
| **2** | *E.coli* | 1.79 | 0 | 1.79 | 2.94 |
| | *P. aeruginosa* | 3.28 | 0 | 3.28 | 4.53 |
| 7 | *E.coli* | 3.65 | 0 | 3.65 | 4.73 |
| | *P. aeruginosa* | 2.23 | 0 | 2.23 | 4.53 |

The combination of zinc lactate and glyceryl caprylate showed synergistic effects for all microorganisms tested on day 2 and day 7. The measured log reduction was higher compared to the expected summation of log reduction and can be seen as a synergistic effect.

In another experiment, an oil-in-water emulsion comprising the combination of zinc lactate and pentylene glycol was incubated with *E.coli* and *P. aeruginosa* and the microbial count reduction was measured after 2 and 7 days. The reduction of colony forming units of bacteria given in logarithmic scale is given in Table 5. The expected log reductions are the sum of the log reductions of both single substances.

**Table 5:**

| **Day** | **bacteria** | **0.5 wt% zinc lactate** | **1.0 wt% pentylene glycol** | **0.5 wt% zinc lactate + 1.0 wt% pentylene glycol** | |
|---|---|---|---|---|---|
| | | | | **expected summation of log reduction** | **measured log reduction** |
| **2** | *E.coli* | 1.79 | 0 | 1.79 | 2.12 |
| | *P. aeruginosa* | 3.28 | 0 | 3.28 | 4.53 |
| **7** | *E.coli* | 3.65 | 0 | 3.65 | 4.73 |
| | *P. aeruginosa* | 2.23 | 0 | 2.23 | 4.53 |

The combination of zinc lactate and pentylene glycol showed synergistic effects for all microorganisms tested on day 2 and day 7 (Table 5).

In another experiment, an oil-in-water emulsion comprising the combination of zinc lactate and caprylhydroxamic acid was tested for *E. coli.* The results are shown in Table 6.

**Table 6:**

| **Day** | **bacteria** | **0.5 wt% zinc lactate** | **0.05 wt% caprylhydroxamic acid** | **0.5 wt% zinc lactate + 0.05 wt% caprylhydroxamic acid** | |
|---|---|---|---|---|---|
| | | | | **expected summation of log reduction** | **measured log reduction** |
| **2** | *E.coli* | 1.79 | 0.14 | 1.93 | 2.94 |
| **7** | *E.coli* | 3.65 | 0.69 | 4.34 | 4.73 |

Caprylhydroxamic acid is a strong chelating agent. The detected synergistic effect in combination with zinc lactate is remarkable because a complexation of zinc does not seem to take place or has no influence to the antimicrobial effect. Synergistic effects have been detected for *E. coli* on day 2 and 7. Thus, a very low dosage of Caprylhydroxamic acid can synergistically boost the antimicrobial efficiency.

In the last experiment, three different microorganisms were tested (*E. coli, P. aeruginosa* and *S. aureus*)*.* A combination of zinc lactate and *Magnolia officinalis* bark extract obtained via extraction with supercritical CO₂ was used. The extract was purified with ethanol. The log reduction was measured after 2 days only (Table 7).

**Table 7:**

| **Day** | **bacteria** | **0.5 % zinc lactate** | **0.2 % *Magnolia officinalis* bark extract** | **0.5 % zinc lactate + 0.2 % *Magnolia officinalis bark extract*** | |
|---|---|---|---|---|---|
| | | | | **expected summation of log reduction** | **measured log reduction** |
| **2** | *E.coli* | 1.79 | 0 | 1.79 | 2.27 |
| | *P. aeruginosa* | 3.28 | 0 | 3.28 | 4.53 |
| | *S. aureus* | 1.46 | 0 | 1.46 | 1.60 |

*Magnolia officinalis* bark extract consists of two antimicrobial ingredients: Magnolol and Honokiol in a ratio of approximately 1:1. Both lignans are highly hydrophobic which leads to challenges for exploitation of their antimicrobial properties in most cosmetic formulations. In water-based formulations they do not dissolve well and in emulsions they migrate to the fat phase where their influence on water-based growth of microorganisms is limited. Zinc lactate is water soluble but does not act as a solvent for Magnolia extract, thus, the detected synergistic effect is unexpected.

In the test sample that solely contains *Magnolia officinalis* bark extract, bacteria were growing and did not show any reduction of colony forming units. Interestingly the combination of zinc lactate and *Magnolia officinalis* bark extract showed synergistic effects on day 2 for *E. coli, P. aeruginosa.* and *S. aureus* (Table 7).

These results are beneficial for skin care products and in particular for oral care products because zinc lactate as well as the actives of *Magnolia officinalis* bark extract Magnolol and Honokiol are highly efficient against caries and periodontitis germs.

In summary, it was shown that zinc lactate has an antibacterial efficiency against *E. coli, P. aeruginosa* and S. *aureus* even at higher pH values. The efficiency can be increased significantly by adding an additional effective ingredient such as glyceryl caprylate, pentylene glycol, caprylyl glycol, caprylhydroxamic acid and *Magnolia officinalis* bark extract.

## Claims

1. Composition comprising at least one zinc salt and at least one additional effective ingredient in an amount achieving a synergistic antimicrobial effect, wherein the additional effective ingredient is selected from:
- glyceryl esters of C₈ to C₁₂ carboxylic acids,
- C₃ to C₁₀ vicinal diols,
- C₆ to C₁₀ hydroxamic acids,
- bi-(hydroxyphenyl) compounds, and/or
- mixtures thereof.

2. Composition of claim 1, wherein the at least one zinc salt is a zinc salt of a carboxylic acid.

3. Composition of claims 1 or 2, wherein the at least one zinc salt is a zinc salt of an alpha hydroxy acid, preferably the zinc salt is zinc lactate.

4. Composition of any of the preceding claims, comprising one zinc salt and one additional effective ingredient in a ratio of 15:1 to 1:3.

5. Composition of any of the preceding claims, wherein at least one additional effective ingredient is a glyceryl ester of a C₈ to C₁₂ carboxylic acid, preferably a glyceryl monoester of a linear C₈ to C₁₂ carboxylic acid, in particular glyceryl caprylate, glyceryl laurate or glyceryl undecylenate.

6. Composition of any of the preceding claims, wherein at least one additional effective ingredient is a C₃ to C₁₀ vicinal diol, preferably a C₅ to C₇ vicinal diol, in particular 1,2-pentanediol or 1,2-hexanediol.

7. Composition of any of the preceding claims, wherein at least one additional effective ingredient is a C₆ to C₁₀ hydroxamic acid, preferably a C₆ to C₁₀ hydroxamic acid with a saturated alkyl chain, in particular caprylhydroxamic acid.

8. Composition of any of the preceding claims, wherein the additional effective ingredient is at least one bi-(hydroxyphenyl) compound, preferably a bi-(hydroxyphenyl) compounds in which each phenyl ring is substituted with one allyl group.

9. Composition of claim 8, wherein the additional effective ingredient is an extract comprising Magnolol and/or Honokiol, preferably the extract is a *Magnolia officinalis* bark extract.

10. Composition of any of the preceding claims, wherein the composition is a cosmetic composition or a personal care formulation.

11. Composition of any of the preceding claims, wherein the at least one zinc salt and at least one additional effective ingredient are present in the composition in an amount effective to achieve at least a 2 log reduction in the number of viable bacteria against the value obtained for the inoculum at a time point of 2 days after inoculation of the composition and/or an amount effective to produce at least a 3 log reduction in the number of viable/live bacteria against the value obtained for the inoculum at a time point of 7 days after inoculation of the composition.

12. Composition of any of the preceding claims, wherein the zinc salt is present in an amount of 0.01 wt% to 4 wt% and/or the additional effective ingredient is present in an amount of 0.01 wt% to 2 wt%, relative to the total weight of the composition.

13. A method of inhibiting microbial growth in a composition comprising:
introducing at least one zinc salt and at least one additional effective ingredient in an amount achieving a synergistic antimicrobial effect, wherein the additional effective ingredient is selected from glyceryl esters of C₈ to C₁₂ carboxylic acids, C₃ to C₁₀ vicinal diols, C₆ to C₁₀ hydroxamic acids, bi-(hydroxyphenyl) compounds, and/or mixtures thereof, and
controlling the growth of at least one microorganism.

14. The method of claim 13, wherein the at least one microorganism is a bacterium from the genus Escherichia, Pseudomonas and/or Staphylococcus, preferably *Escherichia coli, Pseudomonas aeruginosa* and/or *Staphylococcus aureus.*

15. Use of a combination of at least one zinc salt and at least one additional effective ingredient in an amount achieving a synergistic antimicrobial effect for controlling microbial growth, wherein the additional effective ingredient is selected from:
- glyceryl esters of C₈ to C₁₂ carboxylic acids,
- C₃ to C₁₀ vicinal diols,
- C₆ to C₁₀ hydroxamic acids,
- bi-(hydroxyphenyl) compounds, and/or
- mixtures thereof.
